# EUROPÄISCHE PATENTSCHRIFT

(11) **EP 0 443 630 B1**
(45) Veröffentlichungstag und Bekanntmachung des Hinweises auf die Patenterteilung: **29.12.1993**
(21) Anmeldenummer: 91103352.0
(22) Anmeldetag: 25.06.1988
(51) Int. Cl.: A61M 5/158, A61M 5/32

(54) **Kanüle**
Cannula
Canule

(30) Priorität: 10.07.1987 DE 3722800
(43) Veröffentlichungstag der Anmeldung: 28.08.1991
(62) Teilanmeldung aus: 88110181.0
(73) Patentinhaber: B. Braun Melsungen AG, 34209 Melsungen (DE)
(72) Erfinder: Haindl, Hans, Dr., W-3508 Melsungen (DE)
(74) Vertreter: Selting, Günther, Dipl.-Ing.

(56) Entgegenhaltungen:
- US-A- 2 409 979
- US-A- 2 711 733
- US-A- 2 746 454
- US-A- 2 748 769

## Beschreibung

Die Erfindung bezieht sich auf eine Kanüle nach dem Oberbegriff des Anspruches 1.

Eine solche Kanüle ist aus US-A-2 746 454 bekannt. Dabei handelt es sich um Nadeln, deren Einführung in die Vene ohne Verletzungsgefahr der gegenüberliegenden Gefäßwand dadurch erleichtert werden soll, daß der vordere Einstechteil der Kanüle Hakenform erhält, wobei die der verhältnismäßig kurzen Lumenöffnung gegenüberliegende stark gekrümmte Rückenfläche als Gleitfläche wirksam ist. Die vordere runde Kante des Einstechteils ist quer zur Längsachse des Kanülenrohres zur Seite gerichtet und liegt in der Flucht der Außenfläche des Kanülenrohres. Sie ist frei von einem flachen Anschliff, während der hintere Rand der Lumenöffnung zur Bildung einer inneren Schneide flach angeschliffen ist. Eine solche Kanüle wird zur Ausnutzung ihrer gebogenen Gleitfläche auf einer gekrümmten Bahn in ein Blutgefäß eingeführt, deren Verlauf etwa dem Radius der Krümmung der Gleitfläche des Einstechteiles entspricht. Diese durch die hakenförmige Biegung erzwungene winklige Anstellung der Kanüle bei der Punktion führt dazu, daß die scharfe hintere Schneide der Lumenöffnung in diese hineindrängendes Material der zu durchstechenden Fläche ausstanzt, wodurch das Punktionsloch vergrößert wird. Dies ist bei Gewebepunktionen wegen der Traumatisierung nachteilig. Unerwünscht ist der Ausstanzeffekt jedoch auch, wenn die Kanüle zum Nachfüllen von Medikamenten-Applikationsvorrichtungen mit einer implantierten Kapsel verwendet wird, die einen Hohlraum zur Aufnahme des Medikamentes aufweist, an den ein Katheter angeschlossen ist. Das freie Ende des Katheters ist zu einer Infusionsstelle geführt und leitet dieser Medikamente zu. Die nach außen (zur Haut des Patienten hin) gerichtete Wand der implantierten Kapsel besteht aus einer perforierbaren Elastomer-Membran, die mit der Kanüle durchstochen wird, um Medikament in den Hohlraum der Kapsel nachzufüllen.

Das wiederholte Einstechen einer Kanüle in die Membran der Kapsel verursacht bisher Undichtigkeiten der Membran dadurch, daß mit der Schneide des Schliffauges Elastomermaterial ausgestanzt wird, so daß Löcher entstehen, die sich nicht mehr von selbst schließen. Nach einer verhältnismäßig geringen Zahl von Einstichen beginnt die Kapsel zu lecken, was den Anwender zum Auswechseln wenigstens der Kapsel der Applikationsvorrichtung zwingen kann. Außerdem führt die Mitnahme von ausgestanzten Materialteilchen in das Medikamentensystem zu einer Kontamination des Medikaments.

Als Nachfüllkanülen verwendet man bisher sogenannte Huber-Kanülen. Bei der derzeit benutzten Ausführungsform ist das ganze Kanülenrohr am vorderen Ende schräg zur Seite abgeknickt und mit einer im wesentlichen parallel zur Längsachse des Kanülenrohres verlaufenden, ringsum angeschliffenen Lumenöffnung am vorderen Ende des abgeknickten Kanülenrohrabschnittes versehen. Die Lumenöffnung befindet sich dabei weit außerhalb der Kontur des geraden Kanülenrohres. Diese Tatsache bewirkt selbst bei ordnungsgemäßem senkrechtem Ansetzen der Huber-Kanüle Ausstanzungen von Materialpartikeln, weil der abgewinkelte vordere Kanülenrohrabschnitt einseitig keilartig durch die Fläche dringt und die dem Keil abgewandte hintere Schneide des Anschliffes der Lumenöffnung seitlich gegen das zu durchdringende Material gedrückt wird und in die Öffnung eingedrungenes Material abschert. Bei unsachgemäßem schrägen Einstechen der Kanülenspitze in die zu punktierende Fläche ergibt sich ein vermehrtes Eindringen von Wandmaterial in die Lumenöffnung mit der Folge, daß noch größere Materialpartien mit der exponierten Schneide des hinteren Schliffauges der Lumenöffnung abgeschert werden.

Bei einer anderen Ausführungsform der Huber-Kanüle mit seitwärts gerichteter Lumenöffnung (US-A-2 409 979) ist die eine Wandseite des Kanülenrohres ohne Krümmung schräg in Richtung der gegenüberliegenden axialen Wandseite abgeknickt. Die die Lumenöffnung umgebende Umrandung ist tangential zur Kanülenrohraußenfläche eben geschliffen und am hinteren Rand mit einer von außen nach innen abfallenden, d.h. äußeren, Schneide versehen. Der Einstechteil, dessen Spitze in der Flucht des Außenumfanges der axialen Wandseite liegt, ist auf der Umrandung der Lumenöffnung mit Facettenschliff versehen. Es hat sich gezeigt, daß auch die abgeknickte Schrägfläche des Kanülenrohres einen einseitigen Keileffekt bei der Punktion einer künstlichen oder natürlichen Fläche bewirkt, der die Ausbildung eines nicht aufgeweiteten, scharfen Schnittes beeinträchtigt und der beim Vorschub der Kanüle Material in die Lumenöffnung drängt, das an der hinteren Schneide abgehobelt und durch Quetschung beschädigt wird. Das Problem beträchtlicher Wandausstanzungen mit der hinteren Schneide eines Schliffauges ergibt sich auch bei einer anderen bekannten Kanüle (DE-OS 30 13 384), die in ihrem vorderen Bereich eine angeschliffene schräge Lumenöffnung aufweist, von deren vorderem Ende eine Spitze abgewinkelt ist. Das äußere Ende der abgewinkelten Spitze liegt hierbei etwa auf der Mittelachse des Kanülenrohres, während die hintere Schneide des Schliffauges sich am hinteren Ende der Lumenöffnung innerhalb der Kontur des Kanülenrohres befindet. Die durch die gegenseitige Ausrichtung der Teile des vorderen Kanülenrohrendes bedingte Führung der Spitze auf einer S-förmigen Kurvenbahn beim Einstich in eine Punktionsfläche führt zu erheblichem Stanzeffekt, der bei der Punktion von künstlichen und natürlichen Wandungen (Membran oder Körpergewebe) gleichermaßen unerwünscht ist.

Der Erfindung liegt die Aufgabe zugrunde, die eingangs erwähnte Kanüle so zu verbessern, daß bei der Punktion einer natürlichen oder künstlichen Wandung Ausstanzungen vermieden werden, um bei Anwendung der Kanüle, z.B. als Spinalkanüle, Gewebeschäden zu unterbinden und bei Anwendung als Nachfüllkanüle einen leckfreien Zustand der implantierten Kapsel einer Medikamenten-Applikationsvorrichtung nach mehreren hundert Punktionen zu gewährleisten.

Diese Aufgabe wird erfindungsgemäß durch die Merkmale des Anspruches 1 gelöst.

Mit der erfindungsgemäßen Kanüle ergibt sich ein verringerter Stanzeffekt bei der Punktion künstlicher oder natürlicher Wandungen, weil die Position der Spitze des Einstechteils in der radialen Zone der axialen Wand gemeinsam mit der gekrümmten Abbiegung der Rückseite des Kanülenrohres bewirkt, daß ohne schädliche Keilwirkung in der zu durchdringenden Wand ein scharf getrennter Schlitz erzeugt wird, der von dem der Lumenöffnung gegenüberliegenden gebogenen Rücken bis auf den Außendurchmesser des geraden Kanülenrohres konzentrisch aufgeweitet wird und der sich nach Herausziehen der Kanüle jedesmal dicht verschließt. Die kombinierten Merkmale erleichtern die für die Unterbindung der Materialabnahme günstige senkrechte Einstichrichtung der Kanüle, und der Einstich bleibt klein, weil die seitliche Versetzung der Spitze des Einstechteils zur Längsmittelachse durch die Krümmung der Abbiegung so ausgeglichen wird, daß sich im Effekt der Einstich einer zentralen Kegelspitze ergibt. Der ohne Seitenabweichungen präzise koaxial erfolgende Durchtritt des Einstechteiles der Kanüle durch den Trennschlitz in lebendem Gewebe verhindert Gewebetraumatisierung und beschleunigt den Heilungsprozeß. Die Durchtrennung der Materialschicht ohne Materialabnahme läßt bis zu mehreren tausend Punktionen der elastischen Membran der implantierten Kapsel einer Medikamenten-Applikationsvorrichtung zu, ohne daß Lecks entstehen. Die Kapsel kann daher solange implantiert bleiben, wie die jeweilige Therapie verlangt.

Der Patient wird durch das Unterbleiben des mehrmaligen subkutanen Einpflanzens einer Kapsel und durch die Verhinderung des Einschleppens von ausgestanzten Partikeln in seinen Körper geschont. Die Therapie wird auf diese Weise zuverlässiger.

Die angeschliffene innere hintere Schneide der Lumenöffnung verläuft in der Flucht der Innenfläche des Kanülenrohres, und die Spitze des Einstechteils liegt entweder in der Flucht der hinteren Schneide oder an irgendeiner Stelle zwischen der gedachten Schneiden-Verlängerung und der Flucht des Außenumfanges des Kanülenrohres.

Diese Kanüle ist herstellungsmäßig günstig, weil durch einmaliges Schleifen des die Lumenöffnung geschlossen umgebenden Randes ausreichende Trennschärfe erreicht wird. Die Spritze ist in bezug auf die axiale Wand des Kanülenrohres genauso angeordnet wie bei der anderen Ausführungsform der Erfindung, und ein Stanzeffekt ist vermieden.

Die angeschliffene hintere Schneide der Lumenöffnung ist vorzugsweise einwärts gerundet. Die Rundung trägt zusätzlich zur Vermeidung von Materialausstanzungen bei, weil sie beim Durchtritt der Kanüle durch die punktierte Fläche das Material wegdrückt und keinesfalls abschabt.

Der Biegewinkel der gekrümmten Abbiegung der einen Wandseite des Kanülenrohres liegt vorteilhafterweise in einem Bereich von 13° ± 3°, vorzugsweise bei 14°. Als Biegewinkel wird der Winkel zwischen der Längsmittelachse des Kanülenrohres und der an die der Lumenöffnung gegenüberliegende gebogene Rückenseite des vorderen Endes des Kanülenrohres angelegten Tangente bezeichnet.

In der Zeichnung ist ein Ausführungsbeispiel der Erfindung schematisch dargestellt.

Es zeigt:

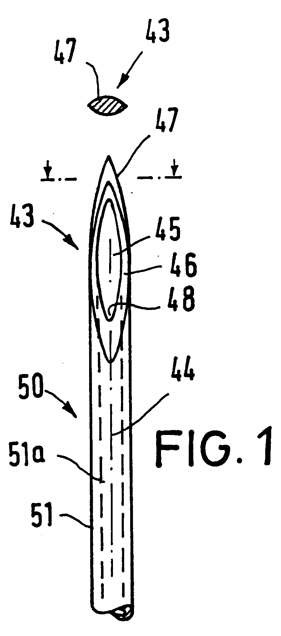

- Fig. 1: eine Draufsicht auf eine Kanüle und einen Querschnitt durch die Spitze.

Die Kanüle 50 besteht aus einem zylindrischen starren Kanülenrohr 51 aus Metall, dessen hinteres Ende z.B. mit einer Injektionsspritze oder einem Schlauch und dergleichen zum Anschluß eines Flüssigkeits-Überleitungssystems verbindbar ist. Das Kanülenrohr 51 ist über den größten Teil seiner Länge gerade. Sein Außendurchmesser beträgt bei dem gezeigten Beispiel 0,7 mm. Der Durchgangskanal kann einen Durchmesser von 0,4 mm aufweisen. Das vordere Ende des Kanülenrohres 51 ist zur Bildung eines Einstechteiles 43 auf einer Wandseite gekrümmt schräg zur Seite abgebogen, während die gegenüberliegende Wandseite 51a axial stehenbleibt, so daß eine ovale Lumenöffnung 45 entsteht, die zur Kanülenrohr-Längsachse 44 etwa parallel verläuft und deren Rand 46 ringsum angeschliffen ist. Die Abbiegung erfolgt im Verlauf der Lumenöffnung 45. Der Anschliff der hinteren Hälfte des Randes 46 bildet ein hinteres Schliffauge mit einer runden Schneide 48, die im Verlauf der Innenfläche der axialen Wandseite 51a liegt. Die Spitze 47 liegt im wesentlichen in der Flucht zwischen zwei gedachten parallelen Linien, die von der Innenfläche und der Außenfläche der axialen Wandseite 51a des Kanülenrohres 51 nach vorne ausgehen. Die Einstech- und Schneidteile des Kanülenrohres 51 liegen innerhalb der Kontur des Kanülenrohres 51 im Bereich der Wandstärke der axialen Wandseite 51a, wodurch bei hervorragender Trenneigenschaft ein Ausstanzen von Partikeln aus der zu durchdringenden natürlichen oder künstlichen Materialschicht verhindert wird. Unter natürlicher Materialschicht wird Gewebe verstanden, das bei Anwendung der Kanüle 50 als Spinalkanüle durchdrungen wird. Eine künstliche Materialschicht wäre z.B. eine Elastomer-Membran bei einer implantierten Kapsel eines Medikamenten-Applikationssystems. Die Länge der Biegung der Rückenseite des Einstechteiles 43 zwischen dem äußeren Punkt der Spitze 47 und der Schneide 48 der Lumenöffnung 45 beträgt bei dem gezeigten Beispiel 3,6 d ± 0,42 d. Sie kann innerhalb der beanspruchten Toleranz länger sein, sofern sie im Schliffverlauf verbleibt. Der Biegewinkel alpha zwischen der Längsmittelachse 44 des Kanülenrohres 51 und der an die gerundet gebogene Rückenseite des Einstechteiles 43 des Kanülenrohres 51 angelegten Tangente beträgt vorzugsweise 13° ± 1°.

Bei dem Einstechteil 43 ist die seitwärts gerichtete, zur Längsachse 44 des Kanülenrohres 51 der Kanüle 50 etwa parallele Lumenöffnung 45 ringsum von einem ebenflächig angeschliffenen Rand 46 umgeben, der den Spitzenbereich 47 freiläßt. Der hintere Rand der Lumenöffnung 45 bildet die angeschärfte innere Schneide 48. Die Schneide 48 kann einwärts gerundet sein. Außerhalb des angeschliffenen Randes 46 hat die Spitze 47 des Einstechteils 43 im Querschnitt die Form des Querschnitts einer bikonvexen Linse mit scharfem Rand.

## Patentansprüche

1. Kanüle mit einem starren Kanülenrohr (51), dessen eine Wandseite am vorderen Ende des Kanülenrohres (51) eine gegen die gegenüberliegende axiale Wandseite gerichtete gekrümmte Abbiegung aufweist, in deren Verlauf in der axialen Wandseite eine Lumenöffnung (45) ausgebildet ist, die zur Kanülenrohrlängsachse etwa parallel verläuft und der ein vorderer Einstechteil (43) und eine hintere Schneide (48) zugeordnet sind,
**dadurch gekennzeichnet**, daß der Einstechteil (43) eine etwa dem Querschnitt einer bikonvexen Linse entsprechende Querschnittsform aufweist und mit einer Spitze (47) versehen ist, die in dem Bereich zwischen zwei gedachten Linien liegt, welche die Innenfläche und die Außenfläche der axialen Wandseite (51a) über die hintere Schneide (48) der Lumenöffnung (45) hinaus nach vorne verlängern.

## Claims

1. Cannula, comprising a rigid cannula tube (51) whose one wall side, at the front end of said cannula tube (51), comprises a curved bend directed towards the opposite axial wall side, in the course of which bend a lumen aperture (45) is formed in the axial wall side, which extends generally parallel to the longitudinal axis of said cannula tube and to which a front piercing member (43) and a rear cutting edge (48) are associated,
**characterized in**
that said piercing member (43) has a cross sectional shape generally corresponding to the cross section of a biconvex lens, and is provided with a tip (47) positioned in the region between two imaginary lines which forwardly elongate the inner surface and the outer surface of said axial wall side (51a) beyond said rear cutting edge (48) of said lumen aperture (45).

## Revendications

1. Canule comportant un tube de canule rigide (51) dont une paroi latérale, située à l'extrémité avant du tube de canule (51), présente une partie courbe dirigée vers la paroi latérale axiale opposée et qui présente sur sa longueur dans la paroi latérale axiale une ouverture (45) qui s'étend à peu près parallèlement à l'axe longitudinal du tube de la canule et sur laquelle sont disposés une partie avant de perçage (43) et un tranchant arrière (48), caractérisée en ce que la partie de perçage (43) présente une forme en section transversale correspondant à peu près à la section transversale d'une lentille biconvexe et comporte une pointe (47) disposée dans la zone située entre deux lignes imaginaires qui prolongent les surfaces interne et externe de la paroi axiale (51a) au-delà du tranchant arrière (48) de l'ouverture (45) vers l'avant.
